Europäisches Patentamt
European Patent Office
Office européen des brevets

(19)

(11) Numéro de publication: 0 192 535
A1

(12) # DEMANDE DE BREVET EUROPEEN

(21) Numéro de dépôt: 86400215.9

(22) Date de dépôt: 31.01.86

(51) Int. Cl.[4]: C 07 D 495/04
A 61 K 31/435
//(C07D495/04, 333:00, 221:00)

(30) Priorité: 31.01.85 FR 8501908

(43) Date de publication de la demande:
27.08.86 Bulletin 86/35

(84) Etats contractants désignés:
AT BE CH DE FR GB IT LI LU NL SE

(71) Demandeur: SANOFI
40, Avenue George V
F-75008 Paris(FR)

(72) Inventeur: Badorc, Alain
1 Rue Lacanal
F-31120 Roquettes(FR)

(72) Inventeur: Frehel, Daniel Résidence l'Autan-Appt. 206
100 Allée de Barcelone
F-31000 Toulouse(FR)

(72) Inventeur: Maffrand, Jean-Pierre
5 Rue du Corps-Franc-Pommiès
F-31120 Portet/Garonne(FR)

(72) Inventeur: Vallee, Eric
253 Chemin du Ramelet Moundi
F-31170 Tournefeuille(FR)

(74) Mandataire: Polus, Camille et al,
c/o Cabinet Lavoix 2, Place d'Estienne d'Orves
F-75441 Paris Cedex 09(FR)

(54) **Nouveaux dérivés de l'acide alpha-]oxo-2-hexahydro-2,4,5,6,7a thiéno (3,2-c)pyridyl-5[ phényl acétique, leur procédé de préparation et leur application thérapeutique.**

(57) La présente invention est relative à de nouveaux dérivés de l'acide α-[oxo-2 hexahydro-2,4,5,6,7,7a thiéno (3,2-c) pyridyl-5] phényl acétique répondant à la formule générale suivante

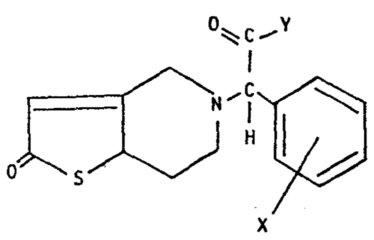

leurs sels d'addition avec les acides minéraux ou organiques pharmaceutiquement acceptables ainsi que les stéréoisomères et leur mélange.

L'invention est également relative à leur procédé de préparation et à leur application thérapeutique en tant qu'agents inhibiteurs de l'agrégation plaquettaire et antithrombotiques.

EP 0 192 535 A1

L'invention est relative à de nouveaux dérivés de l'acide α-[oxo-2 hexahydro-2,4,5,6,7,7a thiéno (3,2-c) pyridyl-5] phényl acétique, à un procédé pour les préparer et à leurs applications en thérapeutique.

Ces dérivés répondent à la formule générale suivante :

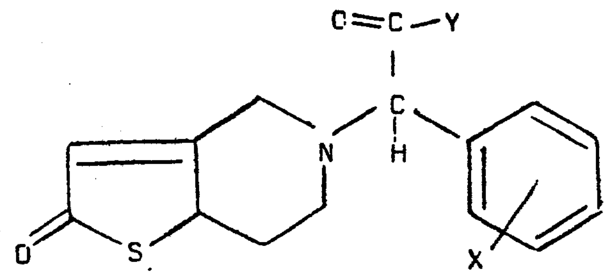

(I)

dans laquelle Y peut représenter l'hydroxyle OH ou le groupe OR dans lequel R peut être un radical alcoyle inférieur linéaire ou ramifié ou un radical aralcoyle éventuellement substitué sur le noyau phényle ou un groupe dialcoylaminoalcoyle de formule :

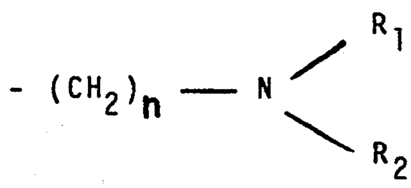

dans lequel n représente un nombre entier de 1 à 4, $R_1$ et $R_2$ sont chacun un radical alcoyle inférieur ou bien $R_1$ et $R_2$ forment ensemble et avec l'atome d'azote auquel ils sont rattachés, un hétérocycle pouvant comporter un second hétéroatome tel que l'oxygène, le soufre ou l'azote, ce dernier pouvant être éventuellement substitué par un radical alcoyle inférieur, ou bien Y représente un groupe amino de formule :

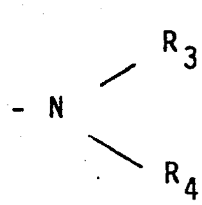

dans lequel $R_3$ et $R_4$ sont chacun indépendamment l'un de

l'autre, l'hydrogène ou un groupe alcoyle inférieur linéaire ou ramifié, saturé ou non, un groupe aryle ou aralcoyle éventuellement substitué, un groupe hétéroaryle ou hétéroaralcoyle, ou bien $R_3$ et $R_4$ forment ensemble avec l'atome d'azote auquel ils sont rattachés, un hétérocycle pouvant comporter un second hétéroatome tel que l'oxygène, le soufre ou l'azote, ce dernier pouvant être substitué par un radical alcoyle inférieur ou phényle éventuellement substitué par au moins un atome d'halogène, un radical alcoyle inférieur, alcoxy inférieur ou trifluorométhyle et X représente l'hydrogène, un halogène, un radical alcoyle inférieur, alcoxy inférieur, trifluorométhyle, nitro, cyano, carboxy ou alcoxycarbonyle.

L'invention comprend aussi des sels d'addition avec les acides minéraux ou organiques pharmaceutiquement acceptables.

Par radical alcoyle ou alcoxy inférieur on entend une chaîne hydrocarbonée saturée en $C_1$-$C_4$.

Par radical aralcoyle on entend un groupe benzyle ou phénéthyle.

Par radical hétéroaryle ou hétéroaralcoyle on entend un groupe (pyridyl-3)méthyle ou (pyridyl-4) méthyle.

Les composés de formule (I) ci-dessus, comportant au moins deux centres asymétriques, peuvent exister sous forme de plusieurs isomères (diastéréoisomères et énantiomères). L'invention concerne donc aussi bien chaque stéréoisomère que leur mélange.

Des dérivés de la thiéno-pyridine ont été décrits dans le brevet N° 2 215 948 et parmi ceux-ci la Ticlopidine douée d'intéressantes propriétés anti-agrégante plaquettaire et anti-thrombotique, a fait l'objet de nombreuses études (HAEMOSTASIS, Vol. 13 supplément 1, 1983).

L'invention a également pour objet un procédé de préparation des composés de formule (I) ci-dessus, caractérisé en ce que l'on prépare les acides, les esters ou les amides de l'invention, dans lesquels Y représente respectivement un groupe hydroxy OH, un groupe alcoxy OR ou un groupe amino - 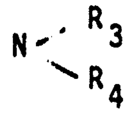 tels que définis ci-dessus, par condensation de la tétrahydro-5,6,7,7a 4H-thiéno (3,2-c) pyridone-2 de formule (II) :

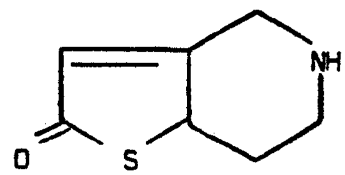

(II)

avec un acide α-halogéno-phényl acétique de formule (IIIa)

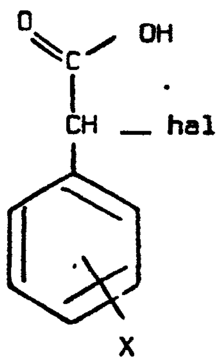

(IIIa)

avec un α-halogéno-phénylacétate de formule (IIIb) :

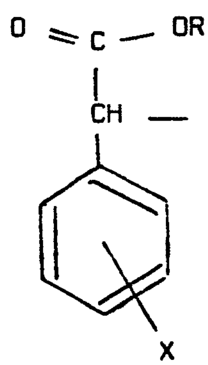

(IIIb)

ou avec un α-halogèno-phénylacétamide de formule (IIIc) :

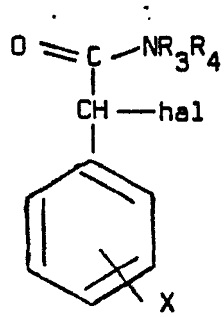

(IIIc)

dans lesquelles hal est un halogène, notamment le chlore, l'iode et le brome, et X, OR et $NR_3R_4$ prennent les valeurs définies ci-dessus, selon le schéma réactionnel :

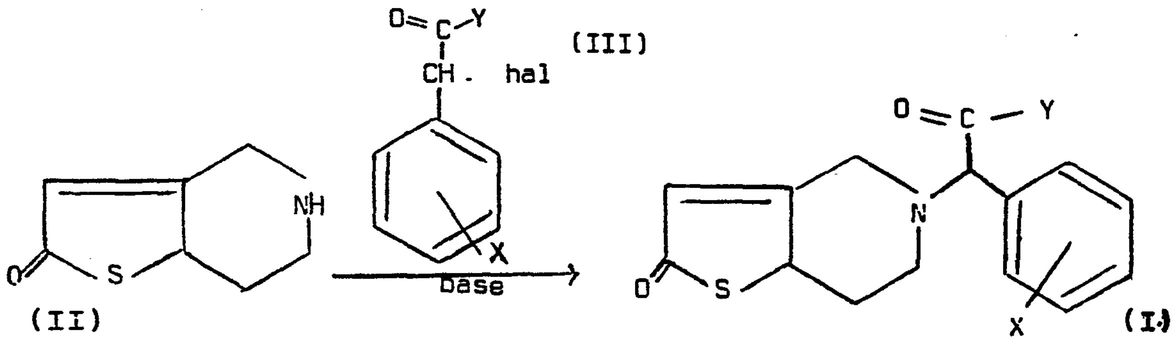


(III) (II) (I)

Y pouvant être l'hydroxyle OH, le radical alcoxy OR ou amino $NR_3R_4$.

Cette condensation s'effectue en présence d'une base faible, notamment un hydrogénocarbonate de métal alcalin tel que le bicarbonate de sodium ou de potassium, dans un solvant inerte tel que le diméthylformamide, le tétrahydrofuranne ou le diméthoxy-1,2-éthane, à des températures comprises entre 40 °C et le point d'ébullition du solvant. Les acides α-halogéno phénylacétiques (IIIa) et les esters α-halogénés de formule (IIIb) sont préparés selon des méthodes connues : (par exemple, E.L. ELTEL, M.T. FISK et T. PROSSER, Organic Syntheses, Coll. Vol. IV, J. WILEY and Sons Inc., New-York, 1963, p. 169).
Les amides α-halogénées de formule (IIIc) sont préparées selon des méthodes connues (par exemple, J. Malcolm BRUCE et F.K. SUTCLIFFE, J. Chem. Soc., 1957, p.4789),

La nouvelle tétrahydro-5,6,7,7a 4H-thiéno (3,2-c) pyridone-2 de formule (II) est obtenue par un procédé consistant :

a) à fixer le groupement protecteur triphénylméthyle (ou trityle) sur la fonction azotée de la tétrahydro-4,5,6,7 thiéno (3,2-c) pyridine donnant le composé de formule (IV) :

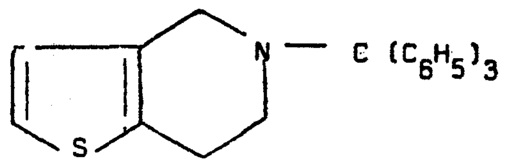

( IV)

b) à fixer le groupe boronique $-B(OR')_2$ en position 2 sur le squelette thiéno(3,2-c) pyridine, par l'intermédiaire du dérivé lithié de formule (V) suivant le schéma réactionnel :

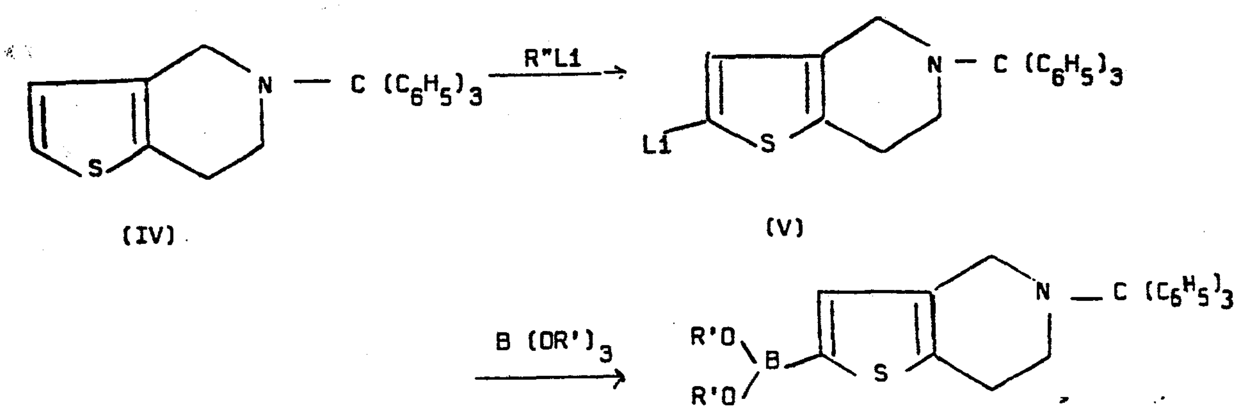


(IV)

(V)

(VI)

c) à oxyder le dérivé boronique de formule (VI) en dérivé borique de formule (VII) :

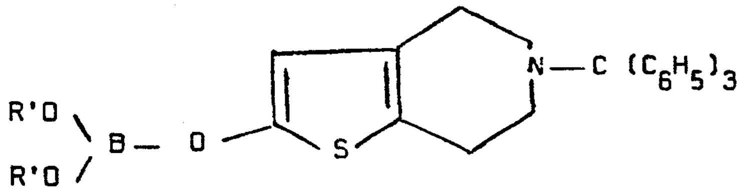

(VII)

d) à hydrolyser immédiatement le composé de formule (VII) en dérivé tritylé de formule (VIII) :

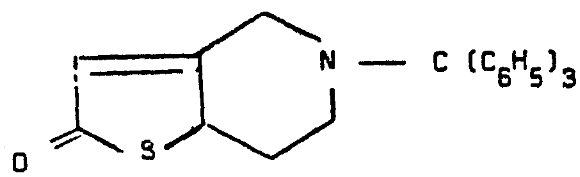

(VIII)

ce composé pouvant exister sous la forme hydroxylée tautomère selon l'équilibre tautomérique :

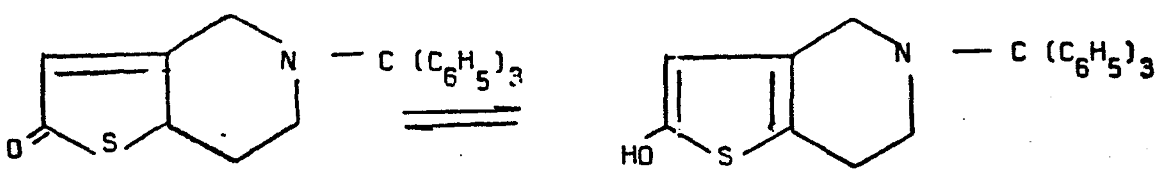

e) à cliver le groupement trityle sélectivement par hydrolyse acide ménagée, pour obtenir la tétrahydro-5,6,7,7a 4H-thiéno (3,2-c) pyridone-2 de formule (II).
Ce composé de formule (II) peut également exister sous la forme hydroxylée tautomère :

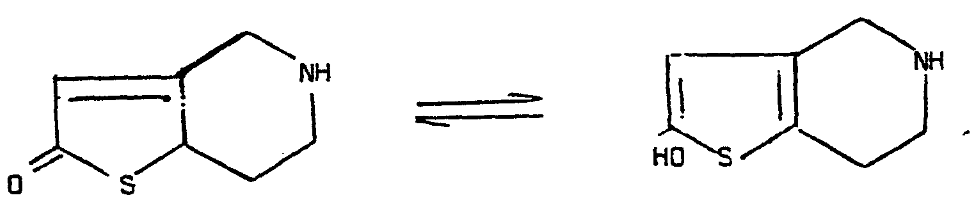

Les trois étapes b) c) d) s'opèrent dans le même réacteur sans isolation des intermédiaires de formules (V), (VI) et (VII).

Le dérivé tritylé (IV) est préparé par condensation du chlorure de triphénylméthyle (trityle) sur la tétrahydro-4,5,6,7 thiéno (3,2-c) pyridine en présence d'une base organique comme accepteur de protons, notamment la triéthylamine, dans un solvant inerte tel que le dichlorométhane, le chloroforme, le tétrahydrofuranne, le diméthoxy-1,2 éthane, le diméthylformamide. Cette condensation s'opère de préférence à température ambiante (20 °C). La lithiation du composé tritylé de formule (IV) avec un alkylithium R"Li tel que le butylithium ou un amidure de lithium tel que le diisopropylamidure de lithium conduit au dérivé lithié de formule (V). Cette métalation s'effectue de préférence entre 0° et 20 °C dans un solvant inerte tel que l'hexane ou le tétrahydrofuranne.

Dans le même réacteur, on condense ce dérivé lithié (V) avec un borate d'alkyle B(OR')3 dans lequel R' est un alkyle inférieur, notamment n-butyle, à des températures comprises entre -20 °C et -40 °C, pour donner le dérivé boronique de formule (VI). Dans le même réacteur, on ajoute une solution aqueuse de 30p.100 d'eau oxygénée pour oxyder le dérivé boronique de formule (VI) en borate de formule (VII) qui s'hydrolyse immédiatement dans le milieu réactionnel, pour donner le dérivé tritylé de formule (VIII). L'hydrolyse acide ménagée du dérivé tritylé de formule (VIII), utilisant l'acide formique à 98 %, l'acide trifluoroacétique ou une solution de gaz chlorhydrique dans l'acétate d'éthyle à des concentrations comprises entre 2 et 5 moles par litre, à des températures comprises entre 40 °C et la température de reflux du milieu réactionnel, permet le clivage sélectif du groupement protecteur trityle, sans clivage du cycle thiophénone, conduisant au composé de formule (II).

Selon une autre variante, les esters et les amides de formule (I) dans laquelle Y est un groupe alcoxy OR ou un groupe amino $-N\begin{matrix} R_3 \\ R_4 \end{matrix}$ (Y, $R_3$ et $R_4$ tels que définis ci-dessus) peuvent être obtenus à partir de l'acide de formule (I) dans laquelle Y est le groupe hydroxyle OH.

S'il est tout à fait possible d'obtenir tous les esters de formule (I) dans laquelle Y est un groupe alcoxy OR

(R tel que défini ci-dessus) par réaction entre les composés de formules (II) et (IIIb), il est préférable, sur le plan économique, de préparer certains d'entre eux et notamment les esters supérieurs, à partir de l'acide de formule (I) dans laquelle Y est le groupe hydroxyle OH et de l'alcool R OH, correspondant, en présence de gaz chlorhydrique ou de chlorure de thionyle, par les méthodes connues, selon le schéma réactionnel :

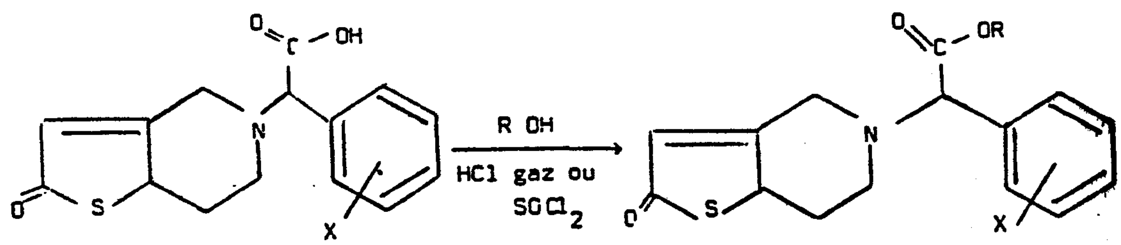


Les amides de formule (I) dans laquelle Y est un groupe amino 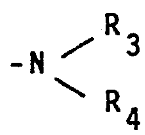 ($R_3$ et $R_4$) tels que définis ci-dessus) ou même certains esters de formule (I) dans laquelle Y est un groupe alcoxy OR (R tel que défini ci-dessus) sont préparés par réaction de l'acide de formule (I) dans laquelle Y est le groupe hydroxyle OH, éventuellement après activation, soit avec l'amine $HNR_3R_4$ ou soit avec l'alcool R OH.

L'activation de l'acide de formule (I) dans lequel Y est le groupe hydroxyle OH peut être obtenue par traitement à l'aide d'un chloroformate d'alcoyle inférieur notamment le chloroformiate d'éthyle ou le chloroformiate d'isobutyle, en présence d'un léger excès de triéthylamine, à des températures comprises entre -5 °C et -10 °C, dans un solvant inerte tel que le chloroforme, le dichlorométhane, le diméthoxy-1,2 éthane ou le tétrahydrofuranne. Il se forme un anhydre mixte de formule (XI) :

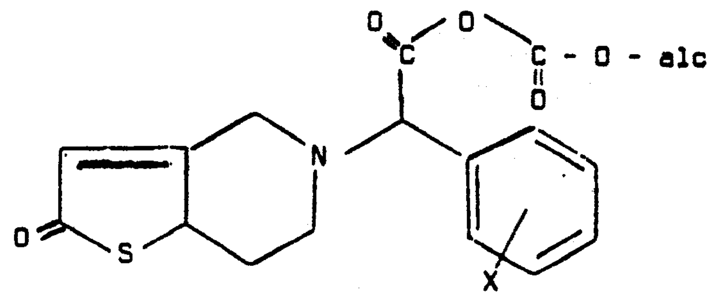

(XI)

(alc pouvant être éthyle, isobutyle notamment) dont le traitement, in situ, par un léger excès, soit de l'amine $HNR_3R_4$, soit de l'alcool R OH, à des températures comprises entre 10 °C et la température ambiante, conduit respectivement aux amides ou aux esters de formule (I) selon le schéma réactionnel :

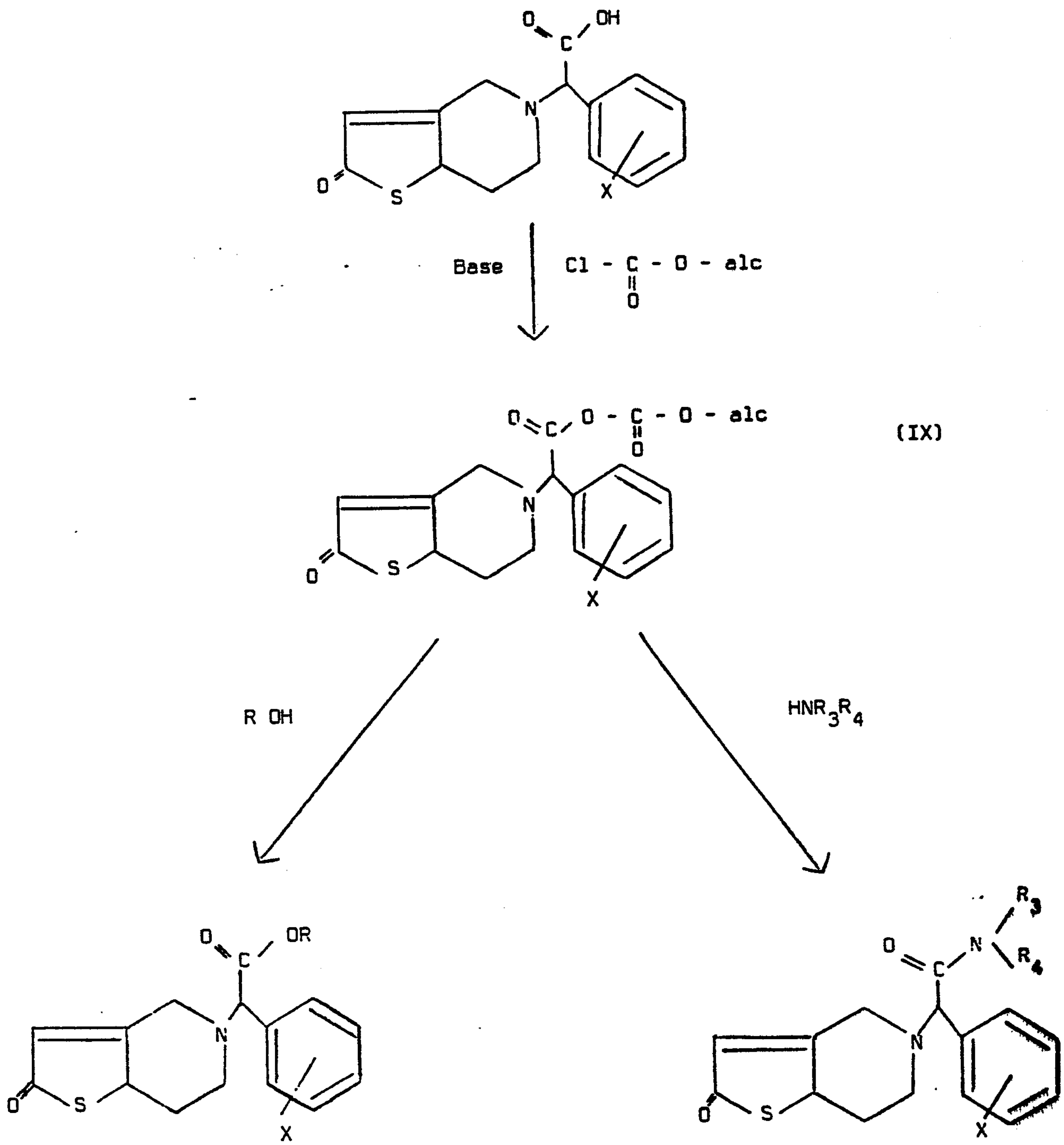


(IX)

L'activation de l'acide de formule (I) dans laquelle Y est un groupe hydroxyle OH, peut aussi être obtenue de différentes manières : ainsi, on a aussi préparé les amides de formule (I) dans laquelle Y est un groupe amino $NR_3R_4$ ($R_3$ et $R_4$ tels que définis ci-dessus) en condensant l'acide de formule (I) dans laquelle Y est un groupe hydroxy OH, avec l'amine $HNR_3R_4$, en présence de dicyclohexylcarbodiimide en solution dans le dichloro-1,2 éthane ou en présence de dicyclohexylcarbodiimide et de N-hydroxybenzotriazole en solution dans le dichlorométhane.

Selon une autre variante, l'acide de formule (I) dans laquelle Y est le groupe hydroxyle OH peut être obtenu par hydrolyse sélective des esters de formule (I) dans laquelle Y est un groupe alcoxy OR (R tel que défini ci-dessus), selon le schéma réactionnel :

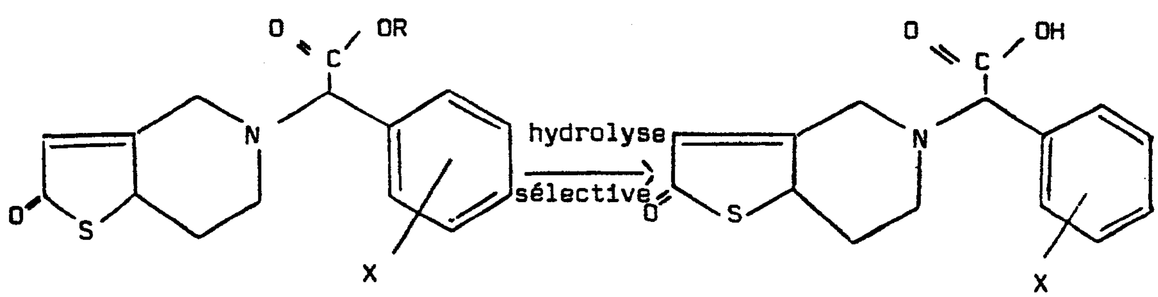


Notamment, l'hydrolyse de l'ester tertiobutylique de formule I (R = tertio-butyle) s'opère dans un milieu acide tel que l'acide trifluoroacétique, l'acide formique à 98 %, à des températures comprises entre 5 °C et le point d'ébullition du milieu réactionnel.

Les exemples, non limitatifs suivants, sont donnés à titre d'illustration de l'invention.

EXEMPLE 1

Triphénylméthyl-5 tétrahydro-4,5,6,7 thiéno (3,2-c) pyridine (IV)

A une solution de 100 g (0,718 mole) de tétrahydro-4,5,6,7 thiéno (3,2-c) pyridine dans 200 ml de dichlorométhane, on ajoute 80 ml (0,789 mole) de triétylamine,

puis goutte à goutte 200,2 g (0,718 mole) de chlorure de triphénylméthyle, à température ambiante. On abandonne le milieu réactionnel, pendant une nuit, à température ambiante. Le milieu réactionnel est versé sur 2 000 ml d'eau. La phase organique est décantée, séchée sur sulfate de sodium et évaporée à sec. Le résidu est purifié par une filtration sur un lit de silice (élution avec le dichlorométhane).

Cristaux blancs, F = 95 °C (pâteux), rendement : 96 %

RMN $^1$H (en ppm) ($CDCl_3$) : 7,57-6,90 (m, 15H) ; 6,80 (d, J = 6,5 Hz, 1H) ; 6,43 (d, J = 6,5 Hz, 1H) ; 3,35 (s, 14), 3,00-2,33 (m, 4H)

EXEMPLE 2

Triphénylméthyl-5 tétrahydro-5,6,7,7a 4H-thiéno (3,2-c) pyridine (VIII)

A une solution de 27 g (0,0707 mole) de triphénylméthyl-5 tétrahydro-4,5,6,7 thiéno (3,2-c) pyridine (IV) dans 300 ml de tétrahydrofuranne, on ajoute goutte à goutte, à 0 °C, 5,31 ml de butyl lithium (solution 1,6M dans l'hexane) (0,085 mole). On agite 15 minutes à température ambiante puis refroidit à -20 °C et on ajoute à cette température, goutte à goutte, 23 ml (0,085 mole) de borate de tri-n-butyle dissous dans 50 ml de tétrahydrofuranne. On agite pendant 1 heure à 10 °C. Après refroidissement du milieu réactionnel à -40 °C, on ajoute goutte à goutte 20,1 ml (0,177 mole) d'eau oxygénée à 30 % en volume. On laisse revenir à température ambiante et agite à cette température pendant 1 heure. On ajoute de l'eau au milieu réactionnel et extrait avec du dichlorométhane. La phase organique d'extraction est lavée à l'eau et séchée sur sulfate de sodium. L'évaporation du solvant à sec laisse un résidu qui est cristallisé dans l'éther diisopropylique.

Cristaux beiges, F = 210 °C (déc.), rendement : 64 %

IR (BRr) : $\nu_{C=O}$ : 1675 $cm^{-1}$

RMN $^1$H (en ppm) ($CDCl_3$) ; 7,48-6,96 (m,15H) ; 6,02 (s, 1H) ; 4,08 (m, 1H) ; 3,78-1,43 (m, 6H).

EXEMPLE 3

Tétrahydro-5,6,7,7a 4H-thiéno (3,2-c) pyridone-2 (II)

On chauffe à 90 °C, pendant 1 heure, 56,9 g (0,143 mole) de triphénylméthyl-5 tétrahydro-5,6,7,7a 4H-thiéno (3,2-c) pyridone-2 (VIII) dans 350 ml d'acide formique à 98 %. Après refroidissement, on ajoute un excès d'une solution saturée de gaz chlorhydrique dans l'éther diéthylique et concentre le milieu réactionnel à 100 ml. On ajoute alors 1 000 ml d'éther diéthylique et filtre les cristaux obtenus, que l'on lave avec de l'éther diéthylique. Les cristaux obtenus sont redissous dans l'eau et la solution aqueuse, après traitement au noir animal et filtration sur un lit de célite, est lyophilisée. Les cristaux ainsi obtenus sont lavés successivement avec de l'acétone puis de l'éther diéthylique et séchés.

Cristaux crème, F = 210 °C (déc.), rendement : 81 %

IR (KBr) : $\nu_{C=O}$ : 1650 $cm^{-1}$

RMN $^{1}H$ en ppm ($DMSO-d_6$) : 6,47 (s,1H) ; 4,97-4,58 (m,1H) ; 4,46-3,83 (2d,2H)

EXEMPLE 4

α-(oxo-2 hexahydro-2,4,6,7,7a thiéno (3,2-c) pyridyl-5) (chloro-2-phényl)-acétate de méthyle (I : Y = $OCH_3$ ; X = 2-Cl) (dérivé n°1)

A une solution de 10 g (0,052 mole) de chlorhydrate de tétrahydro-5,6,7,7a 4H-thiéno (3,2-c) pyridone-2 (II) dans 100 ml de diméthylformamide, on ajoute 10,43 g (0,104 mole) de bicarbonate de potassium et 7,82 g (0,052 mole) d'iodure de sodium, puis 11,65 g (0,0532 mole) d' α-chloro (chloro-2 phényl)-acétate de méthyle. (III : Y = $OCH_3$ ; X = 2-Cl ; hal = Cl). On chauffe le milieu réactionnel à 60 °C pendant 90 minutes. Après refroidissement, on verse le milieu réactionnel dans 600 ml d'eau. On extrait avec de l'acétate d'éthyle, lave l'extrait organique avec de l'eau. La phase organique est séchée sur sulfate de sodium. L'évaporation du solvant laisse un résidu que l'on purifie par filtration sur un lit de silice (élution à l'acétate d'é-

thyle). Le produit huileux obtenu est transformé en chlorhydrate, pour l'ultime purification.

Chlorhydrate, cristaux blancs, F = 130 °C (déc.), rendement : 58 %

IR (KBr) : $\nu_{C = O}$ (ester) : 1745 $cm^{-1}$ ; $\nu_{C = O}$ (thiolactone) : 1680 $cm^{-1}$

RMN $^1H$ en ppm (DMSO-$d_6$) : 7,43 (m,4H) ; 6,27 (s,1H) ; 5,33 et 5,25 (s,1H), 2 diastéréoisomères) ; 4,77-4,38 (m, 1H) ; 3,67 (s, 3H).

EXEMPLE 5

α-(oxo-2 hexahydro-2,4,5,6,7,7a thiéno (3,2-c) pyridyl-5) phénylacétate de méthyle (I : Y = $OCH_3$ ; X = H) - dérivé n°2).

Ce composé est préparé selon le mode opératoire décrit dans l'exemple 4 par alcoylation de la tétrahydro-5,6,7,7a 4H-thiéno (3,2-c) pyridone-2 (II) avec l' α-chlorophénylacétate de méthyle (III : Y = $OCH_3$ ; X = H ; Hal = Cl)

Bromhydrate, cristaux blancs, F = 205 °C (déc.), rendement 86 %

IR (KBr) : $\nu_{C = O}$ (ester) : 1745 $cm^{-1}$ ; $\nu_{C = O}$ (thiolactone) : 1695 $cm^{-1}$

RMN $^1H$ en ppm (DMSO-$d_6$) : 7,60 (m,5H) ; 8,57 (s,1H) ; 5,83 (s, 1H) ; 3,77 (s, 3H)

EXEMPLE 6

α-(oxo-2 hexahydro-2,4,6,7,7a thiéno (3,2-c) pyridyl-5) (fluoro-2 phényl)-acétate de méthyle (I : Y = $OCH_3$ ; X = 2-F) dérivé N°3.

Ce composé est préparé selon le mode opératoire décrit dans l'exemple 4 par alcoylation de la tétrahydro-5,6,7,7a 4H-thiéno (3,2-c) pyridone-2 (II) avec l'α -chloro (fluoro-2 phényl)-acétate de méthyle (III ; Y = $OCH_3$ ; x = 2 - F ; hal - Cl)

Bromhydrate, cristaux blancs, F = 213 °C, rendement : 70 %

IR (KBr) : $\nu_{C = O}$ (ester) : 1745 $cm^{-1}$ ; $\nu_{C = O}$ (thiolac-

tone) : 1690 $cm^{-1}$

RMN $^1H$ en ppm (DMSO-d6) : 7,48 (m, 4H) ; 6,43 (s, 1H) ; 5,60 (s, 1H), 4,83-3,87 (m, 3H); 3,72 (s, 3H)

EXEMPLE 7

α-(oxo-2 hexahydro-2,4,6,7,7a thiéno (3,2-c) pyridyl-5) (chloro-2 phényl)-acétate d'éthyle (I : Y = $OC_2H_5$ ; X = 2-Cl) dérivé N°4.

Ce composé est préparé selon le mode opératoire décrit dans l'exemple 4 par alcoylation de la tétrahydro-5,6,7,7a 4H-thiéno (3,2-c) pyridone-2 (II) avec l'α -chloro (chloro-2-phényl)-acétate d'éthyle (III : Y = $OC_2H_5$ ; X = 2-Cl ; hal = Cl)

Bromhydrate, cristaux blancs, F = 200 °C, rendement : 64 %

IR (KBr) : $\nu_{c=o}$ (ester) : 1755 $cm^{-1}$ ; $\nu_{c=o}$ (thiolactone) : 1680 $cm^{-1}$

RMN $^1H$ en ppm (DMSO-d6) ; 7,52 (m,4H) ; 6,53 (s,1H) ; 5,78 (s,1H) ; 4,30 (m,2H), 1,18 (t,J = 7Hz, 3H)

EXEMPLE 8

α-(oxo-2-hexahydro-2,4,5,6,7,7a thiéno (3,2-c) pyridyl-5) (méthyl-2 phényl)-acétate d'éthyle (I : Y = $OC_2H_5$ ; X = 2-$CH_3$) dérivé N°5

Ce composé est préparé selon le mode opératoire décrit dans l'exemple 4 par alcoylation de la tétrahydro-5,6,7a 4H-thiéno (3,2-c) pyridone-2 (II) avec l' α-chloro (méthyl-2 phényl)-acétate d'éthyle (III : Y = $OC_2H_5$ ; X = 2-$CH_3$ ; hal = Cl)

Bromhydrate, cristaux blancs, F = 222 °C (déc.), rendement: 79 %.

IR (KBr) = $\nu_{c=o}$ (ester) : 1745 $cm^{-1}$ ; $\nu_{c=o}$ (thiolactone); 1685 $cm^{-1}$

RMN $^1H$ en ppm (DMSO-d6) : 7,38 (m,4H) ; 6,60 (s,1H) ; 5,60 (s,1H) ; 4,23 (m,2H) ; 2,53 (s,3H) ; 1,13 (t. J = 7Hz, 3H).

EXEMPLE 9

α-(oxo-2 hexahydro-2,4,5,6,7,7a thiéno (3,2-c) pyridyl-5) (chloro-2 phényl)-acétate d'isopropyle (I : Y = OCH $(CH_3)_2$ ; X = 2-Cl) dérivé N°6.

Ce composé est préparé selon le mode opératoire décrit dans l'exemple 4 par alcoylation de la tétrahydro-5,6,7,7a 4H-thiéno (3,2-c) pyridone-2 (II) avec l' α-chloro (chloro-2 phényl)-acétate d'isopropyle (III, Y = $OCH(CH_3)_2$ ; X = 2-Cl, Hal = Cl)

Hémisulfate, cristaux beiges, F = 110 °C, rendement : 69 %

IR (KBr) : $\nu_{C=O}$ (ester) : 1750 $cm^{-1}$ : $\nu_{C=O}$ (thiolactone) : 1690 $cm^{-1}$

RMN $^1H$ en ppm (DMSO-d6) : 7,50 (m,4H) ; 6,36 et 6,28 (s, 1H,2 diastéréoisomères 5,36 et 5,28 (s,1H,2 diastéréoisomères : 1,33-0,87 (m,6H)

EXEMPLE 10

α-(oxo-2 hexahydro-2,4,5,6,7,7a thiéno (3,2-c) pyridyl-5) (chloro-2 phényl)-acétate de tertiobutyle (I : Y = $OC(CH_3)_3$ X = 2-Cl) dérivé N°7

Ce composé est préparé selon le mode opératoire décrit dans l'exemple 4 par alcoylation de la tétrahydro-5,6,7,7a 4H thiéno (3,2-c) pyridone-2 (II) avec l' α-chloro (chloro-2 phényl)-acétate de tertiobutyle.

(III : Y = $OC(CH_3)_3$ ; $H_2$ = 2-Cl ; Hal = Cl)

Base huile, rendement : 69 %

IR (film) : $\nu_{C=O}$ (ester) : 1740 $cm^{-1}$ ; $\nu_{C=O}$ (thiolactone) : 1690 $cm^{-1}$

RMN $^1H$ en ppm (DMSO-d6): 7,36 (m, 4H) ; 6,02 (s,1H) ; 4,63 (s 1H) ; 4,76-4,13 (m, 1H) ; 1,40 (m,9H).

EXEMPLE 11

N-[α-(oxo-2 hexahydro-2,4,5,6,7,7a thiéno (3,2-c) pyridyl-5) (chloro-2 phényl) ]-acétyl pyrrolidine (I : Y =

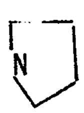 ; X = 2 Cl) dérivé N° 8

A une solution de 4,5 g (0,023 mole) de chlorhydrate de tétrahydro-5, 6,7,7a 4H-thiénol (3,2-c) pyridone-2 (II) dans 45 ml de diméthylformamide, on ajoute 4,7 g (0,047 mole) de bicarbonate de potassium puis 3,5 g (0,023 mole) d'iodure de sodium. A ce milieu réactionnel on ajoute 6,1g (0,023 mole) d' -chloro (chloro-2 phényl)-acétyl pyrrolidine (III ; Y = N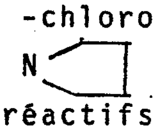 ; X = 2-Cl ; hal = Cl) et porte l'ensemble des réactifs à 60 °C pendant deux heures. Après refroidissement, on ajoute de l'acétate d'éthyle. La solution organique est lavée avec de l'eau puis séchée sur du sulfate de sodium. On évapore à sec et le résidu est purifié par filtration sur un lit de silice (éluant : acétate d'éthyle). L'ultime purification s'opère par transformation en chlorhydrate dans l'éther éthylique.

Chlorhydrate, cristaux blancs, F = 150 °C, rendement : 69 %

IR (KBr): $\nu_{C = O}$ (thiolactone) : 1690 $cm^{-1}$ ; $\nu_{C = O}$ (amide) : 1650 $cm^{-1}$

RMN $^1H$ en ppm ($CDCl_3$) : 7,31 (m, 4H) ; 5,90 (s, 1H) ; 4,90 (s, 1H).

EXEMPLE 12

<u>N,N-diméthylα-(oxo-2 hexahydro-2,4,5,6,7,7a thiéno (3,2-c) pyridyl-5) (chloro-2 phényl)-acétamide</u> (I : Y = $N(CH_3)_2$ ; X = 2-Cl) dérivé N°9.

Ce composé est préparé selon le mode opératoire décrit dans l'exemple 11 par alcoylation de la tétrahydro-5,6,7,7a 4H thiéno(3,2-c) pyridone-2 (II) avec l'α-chloro N,N-diméthyl (chloro-2-phényl)-acétamide (III : Y = $N(CH_3)_2$; X = 2-Cl ; Hal = Cl)

Chlorhydrate, cristaux blancs, F = 145 °C, rendement : 73 %.

IR (KBr) : $\nu_{C = O}$ (thiolactone : 1690 $cm^{-1}$ ; $\nu_{C = O}$ (amide) ; 1655 $cm^{-1}$

RMN $^1H$ en ppm (DMSO-d6) : 7,70 (m,4H) ; 6,08 et 6,00 s,1H,2 diastéréoisomères) 2,93 (s,6H).

EXEMPLE 13

<u>Acide α-(oxo-2 hexahydro-2,4,5,6,7,7a thiéno (3,2-c)</u>

<u>pyridyl-5) (chloro-2 phényl)-acétique</u> (I : Y = OH ; X = 2 Cl) dérivé N° 10

a) <u>Alkylation directe de la tétrahydro-5,6,7,7a 4H-thiéno (3,2-c) pyridone-2 (II)</u>

A une solution de 1g (0,00522 mole) de chlorhydrate de tétrahydro-5,6,7,7a 4H-thiéno (3,2-c) pyridone-2 (II) dans 15 ml de diméthylformamide sec, on ajoute 1,09 g (0,011 mole) de bicarbonate de potassium et 0,8 g (0,00522 mole) d'iodure de sodium, puis 1,07 g (0,00522 mole) d'acide $\alpha$-chloro (chloro-2 phényl)-acétique (III : $R_1$ = OH ; $R_2$ = 2-Cl ; X = Cl) et on porte à 60 °C pendant 2 h 30. On verse le milieu réactionnel dans un excès d'acide chlorhydrique 1N. On filtre le précipité, le lave avec de l'eau, puis de l'acétone et de l'éther diéthylique. On sèche à l'étuve les cristaux beiges obtenus.

Rendement : 60 %

b) <u>Hydrolyse sélective d'ester</u>

On agite à température ambiante, pendant une nuit, une solution de 9g (0,024 mole) de $\alpha$-(oxo-2 hexahydro-2,4,5,6,7,7a thiéno (3,2-c) pyridyl-5) (chloro-2 phényl)-acétate de tertiobutyle (I ; Y = $OC(CH_3)_3$ ; X = 2-Cl) dans 50 ml d'acide trifluoroacétique. On évapore à sec et cristallise le résidu dans l'éther diisopropylique. Les cristaux beiges obtenus (F $\simeq$ 100 °C, fusion pâteuse) sont dissous dans 100 ml d'acide chlorhydrique 2N. Il y a dissolution puis reprécipitation. Les cristaux sont essorés, lavés avec de l'eau et séchés.

Cristaux beiges, F = 210 °C, rendement : 80 %

IR (KBr) : $\nu_{C=O}$ (acide) : 1725 $cm^{-1}$ ; $\nu_{C=O}$ (thiolactone) ; 1690 $cm^{-1}$.

RMN $^1H$ en ppm ($CF_3$ COOD) : 7,52 (m,4H! ; 6,53 (s,1H) ; 5,80 (s, 1H).

<u>EXEMPLE 14</u>

<u>$\alpha$-(oxo-2 hexahydro-2,4,5,6,7,7a thiéno (3,2-c) pyridyl-5) (chloro-2 phényl)-acétate de (phényl-2-éthyle</u> (I : Y = $OCH_2$ $CH_2C_6H_5$ ; X = 2-Cl)

Ce composé est préparé selon le mode opératoire décrit dans l'exemple 4 par alcoylation de la tétrahydro-5,6,7,7a 4H-thiéno (3,2-c) pyridone-2 (II) avec l'α-chloro (chloro-2-phényl) acétate de (phényl-2 éthyle) (III = Y = $OCH_2CH_2C_6H_5$ ; X = 2-Cl ; hal = Cl)
Chlorhydrate, cristaux beiges, F = 175 °C (acétone) ; rendement : 71 %.
IR (KBr) : $\nu_{c=o}$ (ester) : 1755 $cm^{-1}$ ; $\nu_{c=o}$ (thiolactone) 1690 $cm^{-1}$.
RMN $^1H$ en ppm (DMSO-d6) : 7,80-6,83 (m, 9H, aromatiques) ; 6,28 (s, 1H) 5,46 et 5,28 (s, 1H, 2 diastéréoisomères)

EXEMPLE 15

N - [α-(oxo-2-hexahydro-2,4,5,6,7,7a thiéno (3,2-c) pyridyl-5) (chloro-2-phényl)]-acétyl morpholine (I : Y = 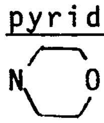 ; X = 2-Cl)

Ce composé est préparé selon le mode opératoire décrit dans l'exemple 11 par alcoylation de la tétrahydro-5,6,7,7a 4H-thiéno (3,2-c) pyridone-2 (II) avec l' α-chloro (chloro-2 phényl)-acétyl morpholine (III : Y = 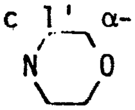 ; X = 2-Cl ; Hal = Cl).
Chlorhydrate, hydrate, cristaux blancs, F = 150°C ; rendement : 60 %
IR (KBr) : $\nu_{c=o}$ (thiolactone) : 1690 $cm^{-1}$ ; $\nu_{c=o}$ (amide): 1660 $cm^{-1}$.
RMN $^1H$ en ppm (DMSO-d6) : 7,51 m, 4H) ; 6,45 et 6,26 (s, 1H, 2 diastéréoisomères) ; 4,86 et 4,73 (s, 1H, 2 diastéréoisomères)

EXEMPLE 16

N-(pyridyl-3-méthyl)α -(oxo-2 hexahydro-2,4,5,6,7,7a thiéno (3,2-c) pyridyl -5) (chloro-2 phényl)-acétamide (I = Y = $NHCH_2$ - 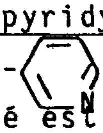 ; X = 2-Cl)

Ce composé est préparé selon le mode opératoire décrit dans l'exemple 11 par alcoylation de la tétrahydro-5,6,7,7a 4H-thiéno (3,2-c) pyridone-2 (II) avec l'α -chloro N-(pyridyl-3-méthyl) (chloro-2 phényl)-acétamide (III : Y =

$NHCH_2$-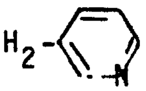 ; X = 2-Cl ; Hal = Cl)

Chlorhydrate, hydrate, cristaux beiges, F = 165 °C ; rendement : 55 %.

IR (KBr) : $\nu_{C=O}$ (thiolactone) : 1690 $cm^{-1}$ ; $\nu_{C=O}$(amide): 1660 $cm^{-1}$.

RMN $^1H$ en ppm (DMSO-d6) : 8,95 - 7,38 (m, 8H) ; 6,38 et 6,32 (s, 1H, 2 diastéréoisomères) ; 5,52 et 5,43 (s, 1H, 2 diastéréoisomères).

EXEMPLE 17

α-(oxo-2 hexahydro-2,4,5,6,7,7a thiéno (3,2-c) pyridyl-5) (méthoxy-4 phényl)-acétate d'éthyle (I : Y = $OC_2H_5$ ; X = 4-$OCH_3$)

Ce composé est préparé selon le mode opératoire décrit dans l'exemple 4 par alcoylation de la tétrahydro-5,6,7,7a 4H-thiéno (3,2-c) pyridone-2 (II) avec l' α-chloro (méthoxy-4 phényl)-acétate d'éthyle (III : Y = $OC_2H_5$ ; X = 4-$OCH_3$ ; Hal : Cl)

Chlorhydrate, cristaux blancs, F = 140 °C ; rendement : 91 %.

IR (KBr) : $\nu_{C=O}$ (ester) ; 1715 $cm^{-1}$; $\nu_{C=O}$ (thiolactone) : 1690 $cm^{-1}$

RMN $^1H$ en ppm (DMSO-d6) : 7,65 (m, 4H) ; 6,53 et 6,61 (s, 1H, 2 diastéréoisomères) ; 5,73 et 5,63 (s, 1H, 2 diastéréoisomères); 3,80 (s, 3H)

EXEMPLE 18

α-(oxo-2 hexahydro-2,4,5,6,7,7a thiéno (3,2-c) pyridyl-5) (chloro-2 phényl)-acétate de [(N,N-diéthylamino)-2 éthyle ] (I : Y = $OCH_2CH_2N(C_2H_5)_2$ ; X = 2-Cl)

Ce composé est préparé selon le mode opératoire décrit dans l'exemple 4 par alcoylation de la tétrahydro-5,6,7,7a 4H-thiéno (3,2-c) pyridone-2 (II) avec l'α-chloro (chloro-2 phényl)-acétate de [(N,N-diéthylamino)-2 éthyle] (III : Y = $OCH_2CH_2N(C_2H_5)_2$ ; X = 2-Cl ; hal = Cl)

Oxalate, cristaux beiges, F = 130 °C ; rendement : 61 %.

IR (KBr) : $\nu_{c=o}$ (ester) : 1745 $cm^{-1}$ ; $\nu_{c=o}$ (thiolactone); 1685 $cm^{-1}$

RMN $^1H$ en ppm (DMSO-d6) : 7,64 - 7,25 (m, 4H) ; 6,23 (s, 1H) ; 4,94 (s, 1H)

EXEMPLE 19

α -(oxo-2 hexahydro-2,4,5,6,7,7a thiéno (3,2-c) pyridyl-5 (chloro-2-phényl)-acétamide (I : Y = $NH_2$ ; X = 2-Cl)

Ce composé est préparé selon le mode opératoire décrit dans l'exemple 11 par alcoylation de la tétrahydro-5,6,7,7a 4H-thiéno (3,2-c) pyridone-2 (II) avec l'α-chloro (chloro-2 phényl)-acétamide (III : Y = $NH_2$ ; X = 2-Cl ; Hal = Cl)

Chlorhydrate, cristaux beiges, F = 185 °C ; rendement : 53 %.

IR (KBr) : $\nu_{c=o}$ (thiolactone) : 1685 $cm^{-1}$ ; $\nu_{c=o}$ (amide) : 1640 $cm^{-1}$

Les résultats pharmacologiques et toxicologiques qui sont rapportés ci-dessous mettent en évidence les propriétés des dérivés de l'invention tant sur le plan de la toxicité et de la tolérance, que sur le plan de leurs activités, notamment inhibitrice de l'agrégation plaquettaire et antithrombotique.

L'invention a donc encore pour objet un médicament présentant en particulier, des activités inhibitrices de l'agrégation plaquettaire et antithrombotique, caractérisé en ce qu'il contient, à titre de principe actif, un dérivé de formule (I) ou un sel d'addition avec un acide minéral ou organique pharmaceutiquement acceptable.

ETUDE TOXICOLOGIQUE

Les composés de l'invention bénéficient d'une excellente tolérance et d'une faible toxicité.

En outre, les essais effectués sur les toxicités aigüe, chronique, sub-chronique et retardée chez différentes espèces animales, n'ont pas permis de mettre en évidence une quelconque réaction locale ou générale, perturbation ou anomalie dans les examens biochimiques, macroscopiques ou microscopiques effectués durant cette expérimentation.

ETUDE PHARMACOLOGIQUE

Cette étude qui a été effectuée comparativement au composé le plus représentatif du brevet cité plus haut, la Ticlopidine, a mis en évidence les actions inhibitrice de l'agrégation plaquettaire et anti-thrombotique des dérivés de l'invention.

i°) Action inhibitrice de l'agrégation plaquettaire

Cette expérimentation est effectuée sur le rat qui a reçu, par la voie orale, X mg des composés de l'invention au moment -2h. Au moment 0h, on prélève 4 ml de sang selon la technique de Renaud, à la veine jugulaire de l'animal anesthésié. C'est ce sang citraté qui est utilisé dans les mesures d'agrégation.

a) mesure de l'agrégation plaquettaire à l'A.D.P.

2 ml de sang citraté sont rapidement versés dans un petit becher placé sur un agitateur magnétique et pourvu d'une barre aimantée. Après quelques secondes d'agitation on introduit dans le bécher 0,4 ml d'une solution contenant 0,66 g d'adénosine-diphosphate (A.D.P) par ml. Après 90 secondes d'agitation, on procède à deux prélèvements de 0,5 ml de sang :

- le premier est mélangé avec 0,5 ml d'une solution EDTA-Formol,
- le deuxième est mélangé avec 0,5 ml d'une solution EDTA seulement.

L'addition d'EDTA-Formol a pour but de stabiliser le sang et donc de fixer l'agrégation tandis que l'EDTA provoque au contraire la désagrégation de tous les amas plaquettaires.

Après un repos de 10 minutes et une centrifugation des 2 mélanges à vitesse lente pendant 5 minutes, afin de séparer les globules rouges, le plasma riche en plaquettes (PRP) surnageant est prélevé, dilué et numéré en plaquettes.

L'intensité de l'agrégation est déterminée par le rapport

$$\frac{\text{nombre de plaquettes dans EDTA-Formol}}{\text{nombre de plaquettes dans EDTA}} \times 100 = \text{pourcentage de plaquettes non agrégées}$$

Le produit à tester est d'autant plus inhibiteur de l'agrégation plaquettaire que le rapport se rapproche de 100.

b) mesure de l'agrégation plaquettaire au collagène

1,5 ml de sang citraté est additionné de 0,10 ml d'une solution contenant 10 µg de collagène par ml. Le milieu étant maintenu en agitation, le comptage des plaquettes est effectué sans interruption.

La diminution du nombre des plaquettes libres en fonction du temps est suivie en continu et permet de tracer une courbe dont la pente donne la vitesse initiale d'agrégation.

Les résultats les plus significatifs sont rassemblés dans le tableau ci-après.

2°) Activité anti-thrombotique

Cette activité a été étudiée selon la méthode de la thrombose veineuse à la vrille.

Elle consiste en une adaptation de la méthode de FRIEDMAN et COLL (AM. J.PHYSIOL., 1980, 199, 770-774). Une spirale métallique (bourre-pâte de dentiste) recoupée est insérée dans la veine cave inférieure du rat qui a reçu 2 heures auparavant, un traitement per os du composé à tester, en suspension dans 10 ml/kg d'une solution aqueuse de gomme arabique à 5 %.

Cinq heures après, cette spirale est prélevée avec le thrombus qu'elle retient, séchée délicatement par tamponnements successifs sur papier filtre et pesée. La spirale est ensuite débarrassée du thrombus, séchée et pesée à nouveau. On obtient ainsi par différence, le poids moyen du thrombus.

Les résultats les plus significatifs sont rassemblés dans le tableau ci-après.

ACTIVITE ANTI-AGREGANTE VIS-A-VIS DE L'ADP

| Composés | Doses orales en mg/kg | % de plaquettes non agrégées | % inhibition | Signification (p) |
|---|---|---|---|---|
| Témoin<br>dérivé N° 1 | 2,5 | 5 ± 1<br>54 ± 12 | 52 | 0,001 |
| Témoin<br>dérivé N° 1 | 5,0 | 6 ± 1<br>96 ± 2 | 96 | 0,001 |
| Témoin<br>dérivé N° 2 | 5,0 | 3 ± 0<br>47 ± 12 | 45 | 0,05 |
| Témoin<br>dérivé N° 3 | 5,0 | 3 ± 0<br>83 ± 2 | 82 | 0,001 |
| Témoin<br>dérivé N° 4 | 5,0 | 5 ± 1<br>49 ± 18 | 46 | 0,05 |
| Témoin<br>dérivé N° 5 | 5,0 | 5 ± 1<br>70 ± 17 | 68 | 0,05 |
| Témoin<br>Ticlopidine | 300,0 | 8 ± 1<br>10 ± 1 | 2 | ns |
| Témoin<br>Ticlopidine | 100,0 x<br>3 jours | 16 ± 4<br>60 ± 7 | 52 | 0,001 |

ACTIVITE ANTI-AGREGANTE VIS-A-VIS DU COLLAGENE

| Composés | Doses orales en mg/kg | Pente d'agrégation | % inhibition | signification (p) |
|---|---|---|---|---|
| Témoin<br>dérivé N° 1 | 2,5 | 1,18 ± 0,11<br>0,11 ± 0,03 | 91 | 0,001 |
| Témoin<br>dérivé N° 1 | 5,0 | 2,48 ± 0,32<br>0 | 100 | 0,001 |
| Témoin<br>dérivé N° 2 | 5,0 | 1,71 ± 0,52<br>0,15 ± 0,10 | 91 | 0,01 |
| Témoin<br>dérivé N° 3 | 5,0 | 1,71 ± 0,52<br>0,08 ± 0,04 | 95 | 0,01 |
| Témoin<br>dérivé N° 4 | 5,0 | 1,94 ± 0,16<br>0,55 ± 0,24 | 66 | 0,01 |
| Témoin<br>Ticlopidine | 300,0 | 10,3 ± 1,17<br>7,94 ± 1,33 | 23 | ns |
| Témoin<br>Ticlopidine | 100,0 x<br>3 jours | 5,1 ± 0,2<br>2,2 ± 0,2 | 56 | 0,01 |

ACTIVITE ANTI-THROMBOTIQUE DANS LE MODELE DE LA VRILLE

| Composés | Doses orales en mg/kg | Poids du Thrombus en mg | % inhibition | Signification (p) |
|---|---|---|---|---|
| Témoin<br>dérivé N° 1 | 12,5 | 3,44 ± 0,35<br>1,49 ± 0,16 | 57 | 0,001 |
| Témoin<br>dérivé N° 1 | 25,0 | 4,82 ± 0,31<br>1,18 ± 0,18 | 76 | 0,001 |
| Témoin<br>dérivé N° 2 | 25,0 | 5,03 ± 0,59<br>1,69 ± 0,16 | 66 | 0,001 |
| Témoin<br>dérivé N° 3 | 25,0 | 3,28 ± 0,25<br>0,94 ± 0,05 | 72 | 0,001 |
| Témoin<br>dérivé N° 4 | 25,0 | 5,25 ± 1,79<br>1,63 ± 0,08 | 69 | 0,001 |
| Témoin<br>dérivé N° 5 | 25,0 | 3,90 ± 0,40<br>1,65 ± 0,14 | 58 | 0,001 |
| Témoin<br>dérivé N° 6 | 25,0 | 3,90 ± 0,40<br>2,63 ± 0,20 | 33 | 0,01 |
| Témoin<br>Ticlopidine | 200,0 | 5,52 ± 0,37<br>5,05 ± 0,46 | 8 | ns |
| Témoin<br>Ticlopidine | 200,0 x<br>3 jours | 4,47 ± 2,03<br>2,03 ± 0,25 | 54 | 0,001 |

Les études toxicologique et pharmacologique qui viennent d'être rapportées ont mis en évidence la faible toxicité des composés de l'invention, ainsi que leur excellente tolérance et leurs intéressantes propriétés inhibitrice de l'agrégation plaquettaire et anti-thrombotique qui les rendent très utiles en thérapeutique humaine et vétérinaire.

Le médicament de l'invention peut être présenté pour l'administration orale sous forme de comprimés, comprimés dragéifiés, capsules, gouttes, granulés ou sirop. Il peut aussi être présenté pour l'administration rectale, sous forme de suppositoires et pour l'administration parentérale, sous forme de soluté injectable.

Chaque dose unitaire contient, avantageusement de 0,005 g à 0,250 g d'un dérivé de l'invention, les doses administrables journellement pouvant varier de 0,005 g à 1,00 g de principe actif en fonction de l'âge du malade et de la sévérité de l'affection traitée.

On donnera ci-après, à titre d'exemples non limitatifs, quelques formulations pharmaceutiques du médicament de l'invention.

1) Comprimés

Dérivé N° 1.................. 0,100 g

Excipient : amidon de maïs, lactose, phosphate dicalcique, stéarate de magnésium

2) Comprimés dragéifiés

Dérivé N°2.................. 0,100 g

Excipient : lévilite, fécule de pomme de terre, acide stéarique, gomme laque, talc, poudre de gomme arabique, sucre cristallisé, cire de carnauba, jaune orangé S.

3) Capsules

Dérivé N° 5................. 0,100 g

Excipient : stéarate de magnésium, lévilite, amidon de maïs, lactose.

4) Soluté injectable

Dérivé N°9.................. 0,075 g

Solvant isotonique q.s.p. 5 ml

5) Suppositoires

Dérivé N° 10................. 0,100 g

Triglycérides semi-synthétiques q.s.p. 1 suppositoire

Par ses propriétés inhibitrices de l'agrégation plaquettaire et anti-thrombotique, le médicament de l'invention est indiqué dans la prévention et le traitement des maladies provoquant une modification pathologique de l'agrégation plaquettaire, telle que les maladies thrombo-emboliques.

## REVENDICATIONS

1 - Composés de formule

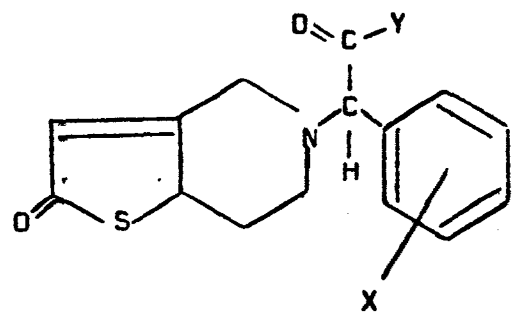

(I)

dans laquelle Y peut représenter l'hydroxyle OH ou un groupe OR dans lequel R peut être un radical alcoyle en $C_1$-$C_4$ linéaire ou ramifié ou un radical benzyle ou phénéthyle éventuellement substitué sur le noyau phényle, ou un groupe dialcoylaminoalcoyle de formule :

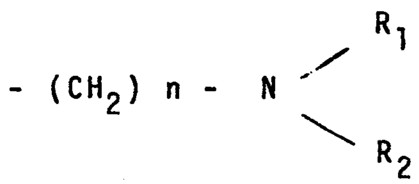

dans lequel n représente un nombre entier de 1 à 4, $R_1$ et $R_2$ sont chacun un radical alcoyle en $C_1$-$C_4$ ou bien $R_1$ et $R_2$ forment ensemble et avec l'atome d'azote auquel ils sont rattachés un hétérocycle pouvant comporter un second hétéroatome tel que l'oxygène, le soufre ou l'azote, ce dernier pouvant être éventuellement substitué par un radical alcoyle en $C_1$-$C_4$, ou bien Y représente encore un groupe amino de formule

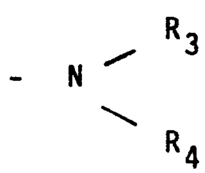

dans lequel $R_3$ et $R_4$ sont chacun indépendamment l'un de l'autre, l'hydrogène ou un groupe alcoyle en $C_1$-$C_4$ linéaire ou ramifié, un groupe aryle ou aralcoyle éventuellement substitué, un groupe hétéroaryle ou hétéroaralcoyle ou bien

$R_3$ et $R_4$ forment ensemble avec l'atome d'azote auquel ils sont rattachés un hétérocycle pouvant comporter un second hétéroatome tel que l'oxygène, le soufre ou l'azote, ce dernier pouvant être substitué par un radical alcoyle en $C_1$-$C_4$ ou phényle éventuellement substitué par au moins un atome d'halogène, un radical alcoyle en $C_1$-$C_4$, alcoxy en $C_1$-$C_4$ ou trifluorométhyle et X représente l'hydrogène, un halogène, un radical alcoyle en $C_1$-$C_4$, alcoxy en $C_1$-$C_4$, trifluorométhyle nitro, cyano, carboxy ou alcoxy ($C_1$-$C_4$) carbonyle, et leurs sels d'addition avec les acides minéraux ou organiques pharmaceutiquement acceptables ainsi que leurs isomères ou leur mélange.

2 - Procédé de préparation des composés selon la revendication 1, caractérisé en ce que :
a) on fixe un groupement trityle sur la fonction azotée de la tétrahydro-4,5,6,7 thiéno(3,2-c) pyridine pour obtenir le composé de formule

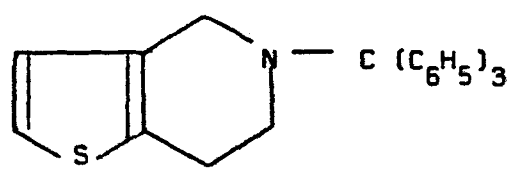

(IV)

b) on fixe le groupement boronique $-B(OR')_2$ dans lequel R' représente un radical alcoyle inférieur pour obtenir le dérivé boronique de formule

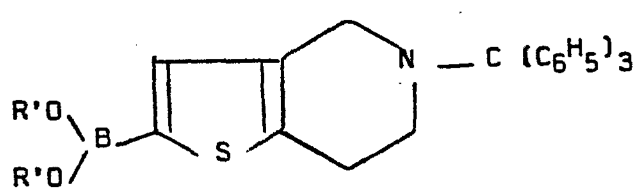

(VI)

c) on oxyde le dérivé boronique (VI) en dérivé borique

de formule

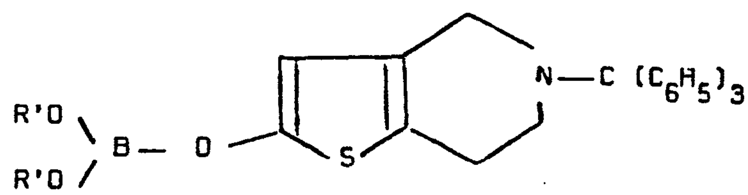

(VII)

dans laquelle R' est un alcoyle en $C_1$-$C_4$

d) on hydrolyse le dérivé borique (VII) pour obtenir le dérivé trityle de formule

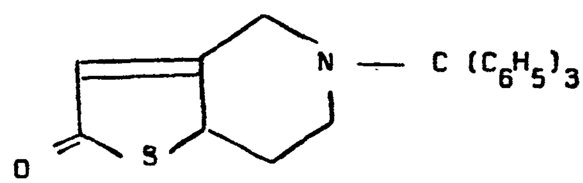

(VIII)

e) on obtient par hydrolyse acide la tétrahydro-5,6,7,7a 4H-thiéno (3,2-c) pyridone de formule

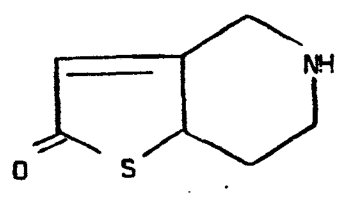

(II)

f) on condense le composé de formule (II) avec un dérivé α-halogéno-phénylacétique de formule (III)

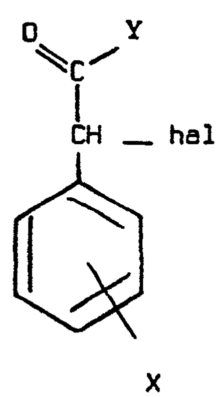

(III)

dans laquelle Y et X prennent les valeurs définies

ci-dessus et hal représente un halogène, notamment le chlore, le brome ou l'iode, ou bien pour préparer l'acide de formule (I) dans lequel Y représente OH, on hydrolyse sélectivement un ester de formüle (I) dans lesquels Y représente OR, mais le cas échéant on transforme l'acide après activation, en amide ou ester de formule (I) par traitement respectivement avec un amine 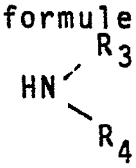 ou avec un alcool R-OH dans lesquels, R, $R_3$ et $R_4$ ont les valeurs définies ci-dessus.

3 - Procédé selon la revendication 2, caractérisé en ce que le dérivé trityle de formule IV est préparé en présence d'une base organique, dans un solvant inerte, à la température ambiante.

4 - Procédé selon la revendication 2, caractérisé en ce que la réaction de condensation entre la thiéno-pyridine de formule (II) et l'ester ou l'amide de formule (III) s'effectue en présence d'une base faible, dans un solvant inerte, à des températures comprises entre 40 °C et la température d'ébullition du solvant.

5 - Procédé selon la revendication 2, caractérisé en ce que l'hydrolyse des esters de formule (I) s'effectue en milieu acide à des températures comprises entre 5 °C et le point d'ébullition du milieu réactionnel.

6 - Procédé selon la revendication 2, caractérisé en ce que l'activation de l'acide de formule (I) est obtenue par le chloroformiate d'alcoyle inférieur, en présence de triéthylamine, dans un solvant inerte, à des températures comprises entre -5 °C et 10 °C.

7 - Procédé selon la revendication 2, caractérisé en ce que l'activation de l'acide de formule (I) pour la préparation des esters est obtenue par le chlorure de thionyle.

8 - Procédé selon la revendication 2, caractérisé en ce que l'activation de l'acide de formule (I) pour la préparation des amides, est obtenue par le dicyclohexylcarbodiimide.

9 - α-(oxo-2 hexahydro-2,4,5,6,7,7a thiéno(3,2-c) pyridyl-5) (chloro-2 phényl)-acétate de méthyle.

10 - α-(oxo-2 hexahydro-2,4,5,6,7,7a thiéno (3,2-c) pyridyl-5) phényl-acétate de méthyle.

11 - α-(oxo-2-hexahydro-2,4,5,6,7,7a thiéno (3,2-c) pyridyl-5) (fluoro-2 phényl)-acétate de méthyle.

12 - α-(oxo-2 hexahydro-2,4,5,6,7,7a thiéno (3,2-c) pyridyl-5) (chloro-2 phényl)-acétate d'éthyle.

13 - α-(oxo-2 hexahydro-2,4,5,6,7,7a thiéno (3,2-c) pyridyl-5) (méthyl-2 phényl)-acétate d'éthyle.

14 - α-(oxo-2 hexahydro-2,4,5,6,7,7a thiéno (3,2-c) pyridyl-5) (chloro-2 phényl)-acétate d'isopropyle.

15 - α-(oxo-2 hexahydro-2,4,5,6,7,7a thiéno (3,2-c) pyridyl-5) (chloro-2 phényl)-acétate de tertiobutyle.

16 - N α-(oxo-2-hexahydro-2,4,5,6,7,7a thiéno (3,2-c) pyridyl-5) (chloro-2 phényl) -acétyl pyrrolidine.

17 - N,N-diméthyl α-(oxo-2-hexahydro-2,4,5,6,7,7a thiéno (3,2-c) pyridyl -5) (chloro-2 phényl)-acétamide.

18 - Acide α-(oxo-2 hexahydro-2,4,5,6,7,7a thiéno (3,2-c) pyridyl-5) (chloro-2 phényl)-acétique.

19 - Médicament caractérisé en ce qu'il contient, à titre de principe actif, un dérivé de formule (I) suivant la revendication 1 ou un sel d'addition avec un acide minéral ou organique pharmaceutiquement acceptable ainsi qu'un des isomères ou leur mélange.

20 - Médicament selon la revendication 19, caractérisé en ce qu'il est présenté sous une forme appropriée à l'administration orale, parentérale ou rectale.

21 - Médicament selon la revendication 19 ou 20, caractérisé en ce qu'il est présenté sous forme de doses unitaires contenant 0,005 g à 0,250 g de principe actif associé à un véhicule pharmaceutiquement acceptable.

## REVENDICATIONS

1 - Procédé de préparation de composés de formule

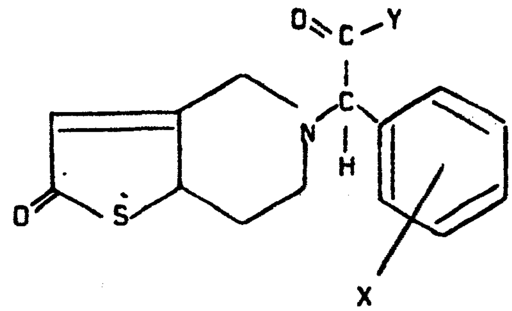

(I)

dans laquelle

Y représente OH ou un groupe OR dans lequel R peut être un radical alcoyle en $C_1$-$C_4$ linéaire ou ramifié ou un radical benzyle ou phénéthyle éventuellement substitué sur le noyau phényle ou un groupe dialcoylaminoalcoyle de formule :

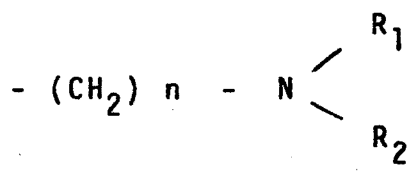

dans lequel n représente un nombre entier de 1 à 4, $R_1$ et $R_2$ sont chacun un radical alcoyle en $C_1$-$C_4$ ou bien $R_1$ et $R_2$ forment ensemble et avec l'atome d'azote auquel ils sont rattachés un hétérocycle pouvant comporter un second hétéroatome tel que l'oxygène, le soufre ou l'azote, ce dernier pouvant être éventuellement substitué par un radical alcoyle en $C_1$-$C_4$, ou bien Y représente encore un groupe amino de formule

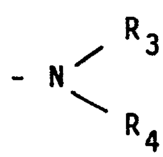

dans lequel $R_3$ et $R_4$ sont chacun indépendamment l'un de l'autre, l'hydrogène ou un groupe alcoyle en $C_1$-$C_4$ linéaire ou ramifié, un groupe aryle ou aralcoyle éventuellement substitué, un groupe hétéroaryle ou hétéroaralcoyle ou bien $R_3$ et $R_4$ forment ensemble avec l'atome d'azote auquel ils

ils sont rattachés un hétérocycle pouvant comporter un second hétéroatome tel que l'oxygène, le soufre ou l'azote, ce dernier pouvant être substitué par un radical alcoyle en $C_1$-$C_4$ ou phényle éventuellement substitué par au moins un atome d'halogène, un radical alcoyle en $C_1$-$C_4$, alcoxy en $C_1$-$C_4$ ou trifluorométhyle et

X représente l'hydrogène, un halogène, un radical alcoyle en $C_1$-$C_4$, alcoxy en $C_1$-$C_4$, trifluorométhyle, nitro, cyano, carboxy ou alcoxy ($C_1$-$C_4$) carbonyle;

ainsi que leurs sels d'addition avec les acides minéraux ou organiques pharmaceutiquement acceptables ainsi que leurs isomères ou leur mélange,

procédé caractérisé en ce que :

a) on fixe un groupement trityle sur la fonction azotée de la tétrahydro-4,5,6,7 thiéno(3,2-c) pyridine pour obtenir le composé de formule

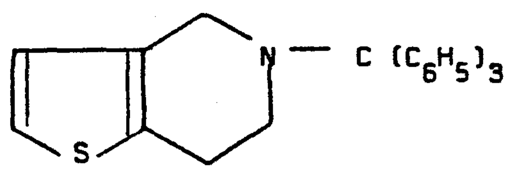

(IV)

b) on fixe le groupement boronique $-B(OR')_2$ dans lequel R' représente un radical alcoyle en $C_1$-$C_4$ pour obtenir le dérivé boronique de formule

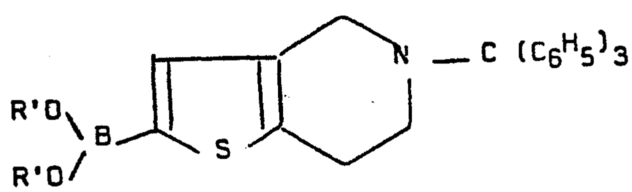

(VI)

c) on oxyde le dérivé boronique (VI) en dérivé borique de formule

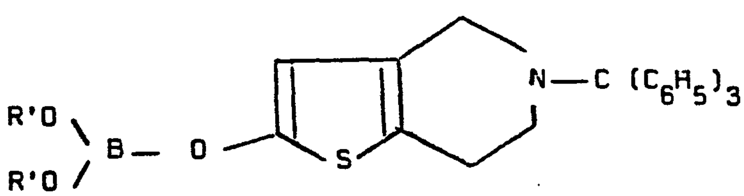

(VII)

dans laquelle R' est un alcoyle en $C_1$-$C_4$,

d) on hydrolyse le dérivé borique (VI) pour obtenir le dérivé trityle de formule

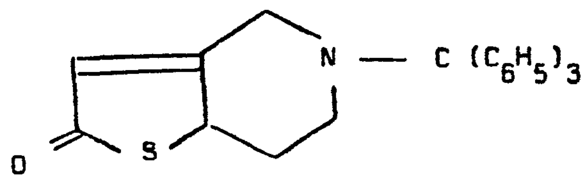

(VIII)

e) on obtient par hydrolyse acide la tétrahydro-5,6,7,7a 4H-thiéno (3,2-c) pyridone de formule

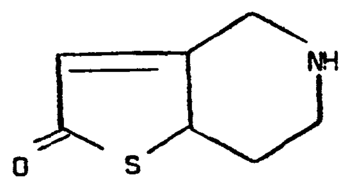

(II)

f) on condense le composé de formule (II) avec un dérivé α-halogéno-phénylacétique de formule (III)

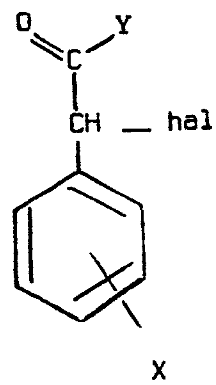

(III)

dans laquelle X et Y prennent les valeurs définies ci-dessus et hal représente un halogène, notamment le chlore, le brome ou l'iode, ou bien pour préparer l'acide de formule (I) dans lequel Y représente OH, on hydrolyse sélectivement un ester de formule (I) dans lesquels Y représente OR, puis le cas échéant, on transforme l'acide, après activation, en amide ou ester de formule (I) par traitement respectivement avec une amine $HN{<}^{R_3}_{R_4}$ ou avec un alcool R-OH dans lesquels R, $R_3$ et $R_4$ ont les valeurs définies ci-dessus.

2 - Procédé selon la revendication 1, caractérisé en ce que le dérivé trityle de formule IV est préparé en présence d'une base organique, dans un solvant inerte, à la température ambiante.

3 - Procédé selon la revendication 1, caractérisé en ce que la réaction de condensation entre la thiéno-pyridine de formule (II) et l'ester ou l'amide de formule (III) s'effectue en présence d'une base faible, dans un solvant inerte, à des températures comprises entre 40 °C et la température d'ébullition du solvant.

4 - Procédé selon la revendication 1, caractérisé en ce que l'hydrolyse des esters de formule (I) s'effectue en milieu acide à des températures comprises entre 5 °C et le point d'ébullition du milieu réactionnel.

5 - Procédé selon la revendication 1, caractérisé en ce que l'activation de l'acide de formule (I) est obtenue par le chloroformiate d'alcoyle inférieur, en présence de triéthylamine, dans un solvant inerte, à des températures comprises entre -5 °C et 10 °C.

6 - Procédé selon la revendication 1, caractérisé en ce que l'activation de l'acide de formule (I) pour la préparation des esters est obtenue par le chlorure de thionyle.

7 - Procédé selon la revendication 1, caractérisé en ce que l'activation de l'acide de formule (I) pour la préparation des amides, est obtenue par le dicyclohexylcarbodiimide.

Office européen des brevets

# RAPPORT DE RECHERCHE EUROPEENNE

0192535

Numéro de la demande

EP 86 40 0215

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 054 442 (SANOFI)<br>* Revendications 1,7 *<br>--- | 1,19 | C 07 D 495/04<br>A 61 K 31/435//<br>(C 07 D 495/04<br>C 07 D 333:00<br>C 07 D 221:00) |
| Y | EP-A-0 099 802 (SANOFI)<br>* Revendications 1,14 *<br>----- | 1,19 | |
| | | | **DOMAINES TECHNIQUES RECHERCHES (Int. Cl.4)**<br>C 07 D 495/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 13-05-1986 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82